# EUROPEAN PATENT APPLICATION

(11) **EP 4 759 927 A1**
(43) Date of publication of application: **17.06.2026**
(21) Application number: 23955286.2
(22) Date of filing: 17.11.2023
(51) Int. Cl.: C12N 9/20, C12N 15/55, C12N 15/70, C12N 1/21, C12P 7/02, C12P 7/40, C12R 1/19

(54) **ESTERASE MUTANT AND APPLICATION THEREOF**

(30) Priority: 13.10.2023 CN 202311325003
(71) Applicant: Tianjin Asymchem Biotechnology Co., Ltd., Tianjin 300457 (CN)
(72) Inventor: HONG, Hao, Morrisville North Carolina 27560 (US); JAMES, Gage, Morrisville North Carolina 27560 (US); ZHANG, Na, Tianjin 300457 (CN); JIAO, Xuecheng, Tianjin 300457 (CN); YANG, Yiming, Tianjin 300457 (CN); YANG, Wenyi, Tianjin 300457 (CN); JIA, Qiqi, Tianjin 300457 (CN); SONG, Zhaoyu, Tianjin 300457 (CN); MOHAMMED LRFAN, Arif, Tianjin 300457 (CN)
(74) Representative: Zacco GmbH
(86) International application number: PCT/CN2023/132411
(87) International publication number: WO 2025/076917

(57) **Abstract**

Provided are an esterase mutant and application thereof. The esterase mutant has a sequence shown in SEQ ID NO:1 with mutation, and the mutation includes I245S; or the esterase mutant includes an I245S mutation, has more than 95% identity with SEQ ID NO:1, and has esterase catalytic activity. The esterase mutant is subjected to a mutation through a site-directed mutagenesis method based on the esterase shown in SEQ ID NO:1, so as to change the amino acid sequence of the esterase, thereby achieving changes in a protein structure and a function, and then the esterase having the above mutation sites is obtained through a directed screening method, such that these esterase mutants have the advantage of greatly-improved enzyme reaction activity and specificity, thereby greatly reducing the amount of enzyme used and the cost of industrial production. Moreover, the esterase has enhanced tolerance to organic solvents, a wide range of reaction temperatures and pH values, and stronger application advantages.

## Description

### Technical Field

The present invention relates to the field of biotechnologies, and in particular, to an esterase mutant and application thereof.

### Background

With the development of pharmaceuticals, pesticides, and other fine chemical industries, organic synthesis faces increasingly greater challenges. For example, due to the chiral recognition capabilities within living organisms, typically only one stereoisomer of a drug molecule has a therapeutic effect, and the other stereoisomers has no therapeutic effect or may even cause side effects. Therefore, in order to synthesize a stereoisomer having a therapeutic effect, traditional organic synthesis often requires the introduction of protection and deprotection steps to compensate for deficiencies in chemical and regioselectivity. However, such operations not only add process flows and increase production costs, but also require the use of a significant number of organic solvents, even including toxic or volatile solvents, thus easily leading to environmental pollution.

Biocatalytic reactions are performed under mild conditions, typically in a neutral manner and at room temperature, or under conditions close to these conditions. In most cases, the biocatalytic reactions occur in aqueous solutions, resulting in minimal environmental pollution. Furthermore, the biocatalytic reactions typically have characteristics of high chemical selectivity, regioselectivity, stereoselectivity, etc., such that unnecessary protection and deprotection steps may be avoided, significantly optimizing production processes, thereby reducing production costs. Therefore, the application of a biocatalytic technology in organic synthesis has significant scientific importance and practical value.

Esterase is collectively referred to a class of enzymes having ability to hydrolyze ester bonds, is widely distributed in animals, plants, and microorganisms, may be applied to organic synthesis, and represents the most extensively studied enzyme in genetic engineering research.

Although the enzyme typically has good reactivity and selectivity toward its natural substrate, its reactivity, stability, and selectivity are generally poor for a non-natural substrate. Generally, genetic modification may be performed through directed evolution, so as to improve the reactivity, stability, and selectivity toward the non-natural substrate, thereby enabling application in industrial production.

### Summary

The present invention aims to provide an esterase mutant and application thereof, so as to improve enzyme reactivity.

In order to achieve the above objective, according to one aspect of the present invention, an esterase mutant is provided. The esterase mutant has the sequence shown in SEQ ID NO:1 with mutation, and the mutation includes I245S; or the esterase mutant includes an I245S mutation, has more than 99% identity with SEQ ID NO:1, and has esterase catalytic activity.

Further, the mutation further includes any one or more of the followings: C33A, A46G, S48N, L65V, T71V, T71M, T87E, L131S, L131N, L131H, R184E, R210Y, K212R, K212M, S230Q, P231T, S235Q, T240F, T240L, T240C, S248D, S248M, K251T, K251L, H262A, H262S, H262C, P336C, or L365M.

Further, the mutation includes any one of the following combined mutations: I245S+C33A, I245S+A46G, I245S+S48N, I245S+L65V, I245S+T71V, I245S+T71M, I245S+T87E, I245S+L131N, I245S+R184E, I245S+R210Y, I245S+K212R, I245S+S230Q, I245S+P231T, I245S+T240F, I245S+T240L, I245S+S248D, I245S+K251T, I245S+K251L, I245S+H262A, I245S+H262S, I245S+P336C, I245S+L365M, I245S+L65V+T71V, I245S+L65V+T71M, I245S+L65V+T87E, I245S+L65V+R210Y, I245S+L65V+K212R, I245S+L65V+P231T, I245S+L65V+T240F, I245S+L65V+T240L, I245S+L65V+K251T, I245S+L65V+L365M, I245S+L65V+H262A, I245S+L65V+H262S, I245S+T71V+C33A, I245S+T71V+L65V, I245S+T71V+T87E, I245S+T71V+L131N, I245S+T71V+T240F, I245S+T71V+T240L, I245S+T71V+K251T, I245S+T71V+H262A, I245S+T71V+H262S, I245S+T71V+L365M, I245S+L65V+H262A+T87E, I245S+L65V+H262A+R210Y, I245S+L65V+H262A+K212R, I245S+L65V+H262A+K212M, I245S+L65V+H262A+T240F, I245S+L65V+H262A+T240L, I245S+L65V+H262A+T240C, I245S+L65V+H262A+S248D, I245S+L65V+H262A+S248M, I245S+L65V+H262A+K251T, I245S+L65V+H262A+K251L, I245S+L65V+H262A+P336C, I245S+L65V+H262A+L365M, I245S+T71V+T240L+C33A, 1245S+T71V+T240L+L65V, I245S+T71V+T240L+T87E, I245S+T71V+T240L+L131N, I245S+T71V+T240L+S235Q, I245S+T71V+T240L+K251T, I245S+T71V+T240L+H262A, I245S+T71V+T240L+H262S, I245S+T71V+T240L+P336C, I245S+T71V+T240L+L365M, I245S+T71V+T240F+L131N+S48N, I245S+T71V+T240L+K251T+L131N, I245S+T71V+T240L+K251T+R210Y, I245S+T71V+T240L+K251T+K212R, I245S+T71V+T240L+K251T+K212M, I245S+T71V+T240L+K251T+S235Q, I245S+T71V+T240L+K251T+H262A, I245S+T71V+T240L+K251T+H262S, I245S+T71V+T240L+K251T+P336C, I245S+T71V+T240L+K251T+L365M, I245S+T71V+T240L+T87E+L65V, I245S+T71V+T240L+T87E+L65V+R210Y, 1245S+T71V+T240L+T87E+L65V+K212M, 1245S+T71V+T240L+T87E+L65V+K251T, I245S+T71V+T240L+T87E+L65V+H262A, I245S+T71V+T240L+T87E+L65V+H262S, I245S+T71V+T240L+T87E+L65V+H262C, I245S+T71V+T240L+T87E+L65V+K251T+K212M, I245S+T71V+T240L+T87E+L65V+K251T+H262A, I245S+T71V+T240L+T87E+L65V+K251T+L365M, 1245S+L65V+H262A+T240F+L131S, I245S+L65V+H262A+T240F+L131H, I245S+L65V+H262A+T240F+L131N, I245S+L65V+H262A+T240F+R210Y, I245S+L65V+H262A+T240F+K212M, 1245S+L65V+H262A+T240F+P231T, I245S+L65V+H262A+T240F+K251T or I245S+L65V+H262A+T240F+L365M.

According to another aspect of the present invention, a DNA molecule is provided. The DNA molecule encodes any one of the above esterase mutants.

According to another aspect of the present invention, a recombinant plasmid is provided. The recombinant plasmid contains any one of the above DNA molecules.

Further, the recombinant plasmid is pET-22a (+), pET-22b (+), pET-3a (+), pET-3d (+), pET-11a (+), pET-12a (+), pET-14b, pET-15b (+), pET-16b (+), pET-17b (+), pET-19b (+), pET-20b (+), pET-21a (+), pET-23a (+), pET-23b (+), pET-24a (+), pET-25b (+), pET-26b (+), pET-27b (+), pET-28a (+), pET-29a (+), pET-30a (+), pET-31b (+), pET-32a (+), pET-35b (+), pET-38b (+), pET-39b (+), pET-40b (+), pET-41a (+), pET-41b (+), pET-42a (+), pET-43a (+), pET-43b (+), pET-44a (+), pET-49b (+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18 or pUC-19.

According to another aspect of the present invention, a host cell is provided. The host cell contains any one of the above recombinant plasmids.

Further, the host cell includes prokaryotic or eukaryotic cells; preferably, the prokaryotic cells are *Escherichia coli* BL21 cells or *Escherichia coli* DH5α competent cells, wherein the eukaryotic cells are yeast.

According to yet another aspect of the present invention, a method for producing a chiral acid is provided. The method includes a step of performing a catalytic reaction on an ester compound using esterase, where the esterase is any one of the above esterase mutants.

Further, an ester compound is 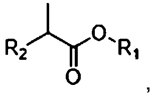 a reaction product is 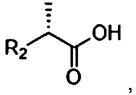 where R1 represents -CH₃, -CH₂CH₃, -CH₂CH₂CH₃, or -CH₂CH₃CH₃; R2 represents one or more of a halogen atom, a nitrogen atom, an oxygen atom, a carbon chain, a heterocycle or a naphthalene ring having different substituents of the halogen atom, nitrogen atom, oxygen atom, and carbon chain; and preferably, the ester compound is

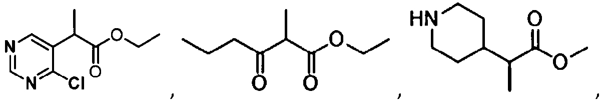

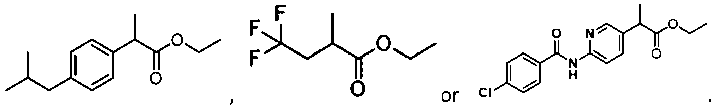

Further, a catalytic reaction of the esterase mutant has a pH of 7.0-9.0 and a reaction temperature of 20-50 °C.

The esterase mutant of the present invention is subjected to a mutation through a site-directed mutagenesis method based on the esterase shown in SEQ ID NO:1, so as to change an amino acid sequence of the esterase, thereby achieving changes in a protein structure and a function, and then esterase having the above mutation sites is obtained through a directed screening method, such that these esterase mutants have the advantage of greatly-improved enzyme reaction activity and specificity, thereby greatly reducing the amount of enzyme used and the cost of industrial production. Moreover, the esterase has enhanced tolerance to organic solvents, a wide range of reaction temperatures and pH values, and stronger application advantages.

### Detailed Description of the Embodiments

It is to be noted that the embodiments in the present application and the features in the embodiments may be combined with one another without conflict. The present invention will be described below in detail with reference to the embodiments.

In the present invention, the specificity of esterase is improved through directed evolution, thereby reducing the amount of the esterase used. A sequence of a template amino acid of the present invention is SEQ ID NO:1 (MQIQGHYELKFEAVRETFAALFDDPQERGAALCIQVGGETVVDLWAGSADKDGQQAWHSDTIAN LFSCTKTFTAVTALQLVGEGKLTLDAPVANYWPEFAQAGKQAITLRQLLSHRAGLPAIRELLPAEALY DWQAMVDALAAETPWWTPGTEHGYAVNTYGWLIGELIRRADGRGPGDSIVARTARPLGLDFHVG LADDEFYRVAHIARGKGNAGDAAAQRLLQVTMREPEALSTRAFTNPPAILTSTNKPEWRRMQQPA HNGHGNARSLAGFYAGLLDGSLLESELLDELTREHSLGQDRTLLTQTRFGLGCMLDQPDVANATF GLGARAFGHPGVGGSVGFADPEHDVAFGFVVNTLGPYILMDPRAQKLVRVLASCL*). A corresponding nucleotide sequence is SEQ ID NO:2 (ATGCAGATCCAAGGCCACTATGAGCTGAAGTTCGAGGCAGTGCGCGAGACGTTCGCAGCGC TGTTCGACGACCCGCAAGAGCGTGGTGCTGCACTGTGTATCCAGGTCGGTGGCGAAACCGTA GTCGACCTGTGGGCAGGTAGCGCAGATAAGGACGGTCAGCAAGCGTGGCATAGCGACACCAT CGCAAACCTGTTCTCCTGCACCAAAACCTTCACCGCGGTTACCGCTCTGCAGCTGGTGGGCG AAGGCAAACTGACCCTGGACGCGCCTGTGGCAAACTACTGGCCAGAATTCGCGCAGGCAGGT AAACAGGCTATCACTCTGCGCCAGCTGCTGAGCCACCGTGCCGGTCTGCCAGCAATCCGTGA ACTGCTGCCGGCTGAAGCACTGTACGACTGGCAAGCAATGGTGGATGCCCTGGCAGCAGAAA CCCCGTGGTGGACTCCGGGTACTGAACATGGTTACGCCGTTAATACCTACGGCTGGCTGATCG GCGAACTGATCCGTCGTGCGGACGGTCGTGGTCCTGGTGACTCTATTGTGGCCCGTACTGCT CGTCCGCTGGGTCTGGATTTCCATGTCGGTCTGGCCGATGACGAATTCTATCGCGTTGCGCAC ATTGCGCGTGGCAAAGGCAACGCGGGCGATGCTGCGGCTCAGCGTCTGCTGCAGGTTACTAT GCGTGAACCGGAAGCTCTGTCTACCCGTGCTTTCACCAACCCGCCGGCGATTCTGACTTCCA CGAACAAACCGGAATGGCGCCGTATGCAGCAGCCGGCGCATAACGGTCACGGTAATGCTCGC TCTCTGGCTGGTTTTTACGCGGGTCTGCTGGACGGTAGCCTGCTGGAATCTGAGCTGCTGGA TGAACTGACGCGCGAACACTCCCTGGGCCAGGATCGCACCCTGCTGACCCAGACTCGCTTTG GTCTGGGTTGTATGCTGGACCAGCCGGATGTTGCTAATGCCACGTTTGGCCTGGGCGCGCGT GCGTTCGGCCACCCAGGCGTGGGCGGCTCTGTTGGCTTCGCTGATCCGGAACACGATGTTG CGTTTGGCTTTGTTGTAAACACCCTGGGTCCGTATATTCTGATGGATCCGCGTGCCCAGAAACT GGTACGTGTACTGGCCTCCTGCCTGTAA). The template of the present invention is a mutant of the esterase derived from *Pseudomonas putida.*

First, a mutation site is introduced on the esterase by means of site-directed mutagenesis, activity and specificity detection is performed on mutants, and the mutants with improved activity and specificity are selected. The I245S mutant shows significantly improved activity and specificity compared to a starting template. Then, a mutant having an I245S mutation is used as a template for continuous mutation, so as to obtain the mutants with improved catalytic activity and specificity.

Herein, the site directed mutation means that a desired change (usually a change representing a favorable direction) is introduced into a target DNA fragment (may be a genome or a plasmid) by a polymerase chain reaction (PCR) and other methods, including base addition, deletion, point mutation and the like. The site directed mutation may quickly and efficiently improve characters and representation of a target protein expressed by DNA, and is a very useful method in gene research.

A method for introducing the site directed mutation using a whole-plasmid PCR is simple and effective, and is a more commonly used method at present. A principle thereof is that a pair of primers (forward and reverse) containing mutation sites are annealed with a template plasmid, and "circularly extended" by a polymerase. The so-called cyclic extension means that the polymerase extends the primer according to the template, and then returns to a 5'-end of the primer to be terminated after one round, and it undergoes circulation of repeated heating and annealing extension, this reaction is different from rolling circle amplification and does not form multiple tandem copies. Extension products of the forward and reverse primers are annealed and matched to form an open-circle plasmid with a nick. The extension product of Dpn I digestion, because the original template plasmid is derived from conventional *Escherichia coli,* is modified by dam methylation, and is chopped because it is sensitive to Dpn I, the plasmid with a mutation sequence synthesized in vitro is not cut up because it is not methylated, so it may be successfully transformed in the subsequent transformation, and a clone of the mutant plasmid may be obtained. The mutant plasmid is transformed into an *Escherichia coli* cell, and then, a crude enzyme is obtained by a method of sonicating cells.

The above mutant plasmid is transformed into an *Escherichia coli* cell, and overexpressed in the *Escherichia coli.* Then, a crude enzyme is obtained by a method of sonicating cells. Best conditions for induction of esterase expression are as follows: it is induced for 16 h at 25 °C and 0.1 mM IPTG.

A typical implementation of the present invention provides an esterase mutant. The esterase mutant has a sequence shown in SEQ ID NO:1 with mutation, and the mutation includes I245S; or the esterase mutant includes an I245S mutation, and has more than 80% identity with SEQ ID NO:1, preferably more than 85%, 86%, 87%, 88%, or 89% identity, and more preferably, more than 95%, 96%, 97%, 98%, or 99%.

Preferably, sites in which amino acid mutation occurs further include any one or more of the followings: C33A, A46G, S48N, L65V, T71V, T71M, T87E, L131S, L131N, L131H, R184E, R210Y, K212R, K212M, S230Q, P231T, S235Q, T240F, T240L, T240C, S248D, S248M, K251T, K251L, H262A, H262S, H262C, P336C, or L365M.

More preferably, the sites in which amino acid mutation occurs include any one of the following combined mutations: I245S+C33A, I245S+A46G, I245S+S48N, I245S+L65V, I245S+T71V, I245S+T71M, I245S+T87E, I245S+L131N, I245S+R184E, I245S+R210Y, I245S+K212R, I245S+S230Q, I245S+P231T, I245S+T240F, I245S+T240L, I245S+S248D, I245S+K251T, I245S+K251L, I245S+H262A, I245S+H262S, I245S+P336C, I245S+L365M, I245S+L65V+T71V, I245S+L65V+T71M, I245S+L65V+T87E, I245S+L65V+R210Y, I245S+L65V+K212R, I245S+L65V+P231T, I245S+L65V+T240F, I245S+L65V+T240L, I245S+L65V+K251T, I245S+L65V+L365M, I245S+L65V+H262A, I245S+L65V+H262S, I245S+T71V+C33A, I245S+T71V+L65V, I245S+T71V+T87E, I245S+T71V+L131N, I245S+T71V+T240F, I245S+T71V+T240L, I245S+T71V+K251T, I245S+T71V+H262A, I245S+T71V+H262S, I245S+T71V+L365M, I245S+L65V+H262A+T87E, I245S+L65V+H262A+R210Y, I245S+L65V+H262A+K212R, I245S+L65V+H262A+K212M, I245S+L65V+H262A+T240F, I245S+L65V+H262A+T240L, I245S+L65V+H262A+T240C, I245S+L65V+H262A+S248D, I245S+L65V+H262A+S248M, I245S+L65V+H262A+K251T, I245S+L65V+H262A+K251L, I245S+L65V+H262A+P336C, I245S+L65V+H262A+L365M, I245S+T71V+T240L+C33A, 1245S+T71V+T240L+L65V, I245S+T71V+T240L+T87E, 1245S+T71V+T240L+L131N, I245S+T71V+T240L+S235Q, I245S+T71V+T240L+K251T, 1245S+T71V+T240L+H262A, I245S+T71V+T240L+H262S, I245S+T71V+T240L+P336C, I245S+T71V+T240L+L365M, I245S+T71V+T240F+L131N+S48N, I245S+T71V+T240L+K251T+L131N, I245S+T71V+T240L+K251T+R210Y, I245S+T71V+T240L+K251T+K212R, I245S+T71V+T240L+K251T+K212M, I245S+T71V+T240L+K251T+S235Q, I245S+T71V+T240L+K251T+H262A, I245S+T71V+T240L+K251T+H262S, I245S+T71V+T240L+K251T+P336C, I245S+T71V+T240L+K251T+L365M, I245S+T71V+T240L+T87E+L65V, I245S+T71V+T240L+T87E+L65V+R210Y, I245S+T71V+T240L+T87E+L65V+K212M, I245S+T71V+T240L+T87E+L65V+K251T, I245S+T71V+T240L+T87E+L65V+H262A, I245S+T71V+T240L+T87E+L65V+H262S, I245S+T71V+T240L+T87E+L65V+H262C, I245S+T71V+T240L+T87E+L65V+K251T+K212M, I245S+T71V+T240L+T87E+L65V+K251T+H262A, I245S+T71V+T240L+T87E+L65V+K251T+L365M, 1245S+L65V+H262A+T240F+L131S, I245S+L65V+H262A+T240F+L131H, I245S+L65V+H262A+T240F+L131N, I245S+L65V+H262A+T240F+R210Y, I245S+L65V+H262A+T240F+K212M, 1245S+L65V+H262A+T240F+P231T, I245S+L65V+H262A+T240F+K251T or I245S+L65V+H262A+T240F+L365M.

The esterase mutant of the present invention is subjected to a mutation through a site-directed mutagenesis method based on the esterase shown in SEQ ID NO:1, so as to change an amino acid sequence of the esterase, thereby achieving changes in a protein structure and a function, and then esterase having the above mutation sites is obtained through a directed screening method, such that these esterase mutants have the advantage of greatly-improved enzyme reaction activity and specificity, thereby greatly reducing the amount of enzyme used and the cost of industrial production. Moreover, the esterase has enhanced tolerance to organic solvents, a wide range of reaction temperatures and pH values, and stronger application advantages.

A typical implementation of the present invention provides a DNA molecule. The esterase encoded by the above DNA improves enzyme activity and enzyme stability, such that the amount of enzyme added may be reduced during the industrial production of amino acids, thereby reducing post-processing difficulty.

The above DNA molecule of the present invention may also exist in the form of an "expression cassette". The "expression cassette" refers to a linear or circular nucleic acid molecule, covering DNA and RNA sequences that may direct the expression of a specific nucleotide sequence in an appropriate host cell. Generally speaking, the expression cassette includes a promoter operatively linked with a target nucleotide, and it is optionally operatively linked with a termination signal and/or other regulatory elements. The expression cassette may also include a sequence required for proper translation of the nucleotide sequence. A coding region usually encodes the target protein, but also encodes a target functional RNA in sense or antisense direction, such as an antisense RNA or an untranslated RNA. The expression cassette containing the target polynucleotide sequence may be chimeric, it means that at least one of its components is heterologous to at least one of the other components thereof. The expression cassette may also be naturally existent, but obtained by efficient recombination for heterologous expression.

A typical implementation of the present invention provides a recombinant plasmid. The recombinant plasmid contains any one of the above DNA molecules. The DNA molecule in the above recombinant plasmid is placed in an appropriate position of the recombinant plasmid, so that the above DNA molecule may be replicated, transcribed or expressed correctly and smoothly.

Although a qualifier used in the present invention to define the above DNA molecule is "containing", it does not mean that other sequences that are not related to its function may be arbitrarily added to both ends of the DNA sequence. It is known by those skilled in the art that in order to meet requirements of a recombination operation, it is necessary to add appropriate digestion sites of a restriction endonuclease at both ends of the DNA sequence, or additionally add a start codon, a stop codon and the like. Therefore, if closed-type expression is used to limit, these situations may not be truly covered.

A term "plasmid" used in the present invention includes any plasmids, cosmids, bacteriophages or agrobacterium binary nucleic acid molecules in double-stranded or single-stranded linear or circular form, preferably a recombinant expression plasmid, or a prokaryotic expression plasmid or a eukaryotic expression plasmid, but preferably the prokaryotic expression plasmid. In some implementations, the recombinant plasmid is selected from pET-22a(+), pET-22b(+), pET-3a(+), pET-3d(+), pET-11a(+), pET-12a(+), pET-14b, pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18, or pUC-19. More preferably, the above recombinant plasmid is pET-22b(+).

A typical implementation of the present invention provides a host cell, and the host cell contains any one of the above recombinant plasmids. The host cell suitable for the present invention includes, but is not limited to, a prokaryotic cell or a eukaryotic cell. Preferably, the prokaryotic cell is an *Escherichia coli* BL21 cell or an *Escherichia coli* DH5α competent cell; and the eukaryotic cell is yeast.

A typical implementation of the present invention provides a method for producing a chiral acid. The method includes a step of performing a catalytic reaction on an ester compound using esterase, where the esterase is any one of the above esterase mutants. The above esterase of the present invention has better specificity or even higher enzyme catalytic activity, such that by using the esterase mutant of the present invention to prepare the chiral acid, production costs can be reduced, and an amino acid ee value obtained is higher.

According to a typical implementation of the present invention, an ester compound is 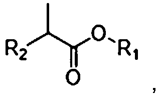 a reaction product is 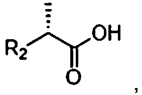 where R1 represents -CH₃, -CH₂CH₃, -CH₂CH₂CH₃, or -CH₂CH₃CH₃; and R2 represents one or more of a halogen atom, a nitrogen atom, an oxygen atom, a carbon chain, a heterocycle or a naphthalene ring having different substituents of the halogen atom, nitrogen atom, oxygen atom, and carbon chain.

Preferably, the ester compound is

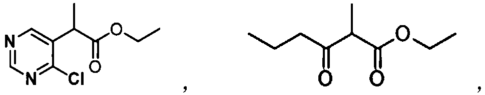

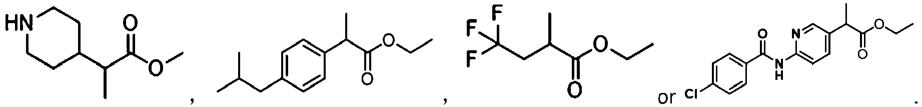

Preferably, a catalytic reaction of the esterase mutant has a pH 7.0-9.0 and a reaction temperature of 20-50 °C.

The beneficial effects of the present invention are further described below with reference to specific embodiments.

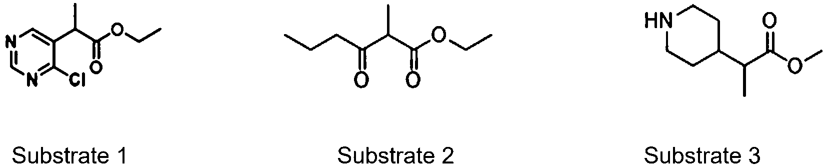

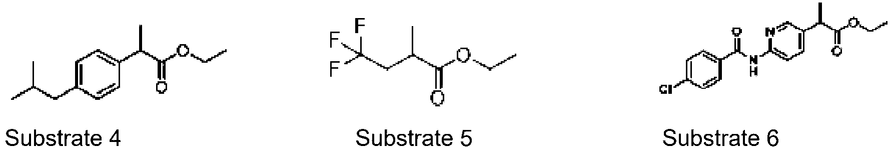

### Embodiment 1

5 mg of a substrate 1, substrate 2, substrate 3, substrate 4, substrate 5, or substrate 6 (dissolved in 0.2 mL DMSO), 5 mg of esterase, and 0.3 M Potassium Phosphate Buffer (KPB) with the pH being 7.5 were added to 1 mL of a reaction system; a reaction was performed for 2 h at 40 °C, and then 3 mL of absolute ethanol was added to 0.5 mL of the reaction system and fully shaken; and centrifugation was performed for 3 min at 12000 rpm, and supernatant liquid was taken for liquid phase detection, so as to detect a conversion rate and an e.e. value. Results were shown in Table 1.

**Table 1**

| Enzyme | Substrate 1 | | Substrate 2 | | Substrate 3 | | Substrate 4 | | Substrate 5 | | Substrate 6 | |
|---|---|---|---|---|---|---|---|---|---|---|---|---|
| | Conversi on rate | e.e . (% ) | Conversi on rate | e.e . (% ) | Conversi on rate | e.e . (% ) | Conversi on rate | e.e . (% ) | Conversi on rate | e.e . (% ) | Conversi on rate | e.e . (% ) |
| Esterase (SEQ ID NO:1) | | 90 | | 40 | | 70 | | 50 | | 40 | | 70 |
| 1245S | + | *** | + | *** | + | *** | + | *** | + | ** | + | ** |
| 1245S+C33 A | + | ** | ++ | ** | ++ | ** | ++ | ** | + | *** | ++ | ** |
| I245S+A46 G | + | ** | + | ** | + | *** | + | *** | ++ | ** | + | *** |
| 1245S+S48 N | - | ** | + | *** | ++ | ** | + | *** | + | *** | + | *** |
| 1245S+L65 V | ++ | *** | + | *** | + | ** | + | *** | ++ | ** | + | *** |
| I245S+T71 V | ++ | *** | ++ | *** | ++ | *** | ++ | *** | ++ | *** | ++ | *** |
| I245S+T71 M | + | *** | + | *** | ++ | ** | ++ | ** | ++ | ** | + | *** |
| 1245S+T87 E | + | *** | + | *** | + | *** | + | *** | + | *** | ++ | ** |
| 1245S+L13 1N | + | *** | + | *** | + | *** | + | *** | ++ | ** | + | *** |
| 1245S+R18 4E | + | *** | + | *** | + | *** | + | *** | + | *** | + | *** |
| 1245S+R21 0Y | ++ | ** | + | *** | ++ | ** | + | *** | + | *** | + | *** |
| 1245S+K21 2R | ++ | ** | + | *** | + | ** | ++ | ** | + | *** | ++ | ** |
| 1245S+S23 0Q | - | ** | + | *** | - | ** | ++ | ** | ++ | ** | + | ** |
| 1245S+P23 1T | + | ** | + | ** | + | *** | - | ** | + | *** | - | ** |
| 1245S+T24 0F | + | *** | + | *** | ++ | ** | + | ** | + | ** | + | *** |
| 1245S+T24 0L | + | *** | + | ** | + | *** | + | *** | ++ | ** | ++ | ** |
| 1245S+S24 8D | - | ** | + | ** | - | ** | + | *** | + | ** | + | *** |
| 1245S+K25 1T | + | * | + | * | + | ** | - | ** | + | ** | - | ** |
| 1245S+K25 1L | + | * | ++ | * | + | *** | + | * | + | * | + | ** |
| 1245S+H26 2A | ++ | ** | ++ | ** | ++ | ** | + | * | ++ | * | + | *** |
| 1245S+H26 2S | ++ | ** | + | ** | + | ** | ++ | ** | + | ** | + | ** |
| 1245S+P33 6C | + | ** | - | *** | + | *** | ++ | ** | + | *** | ++ | ** |
| 1245S+L36 5M | ++ | ** | ++ | ** | ++ | ** | + | *** | ++ | ** | + | *** |

The fold decrease or increase in activity (where the conversion rate represents enzyme activity) relative to a female parent; --- represents a decrease of 10-50 folds; -- represents a decrease of 5-10 folds; - represents a decrease of 1-5 folds; + represents an increase of 1-5 folds; ++ represents an increase of 5-10 folds;+++ represents an increase of 10-50 folds; ++++ represents an increase of more than 50 folds; and +++++ represents an increase of more than 70 folds.
* with an ee value less than 0%; ** with the ee value 0%-0%; *** with the ee value 50%-95%; and **** with the ee value greater than 95%.

### Embodiment 2

A mutation was continuously performed to improve enzyme activity and increase a product ee value.

5 mg of a substrate 1, substrate 2, substrate 3, substrate 4, substrate 5, or substrate 6 (dissolved in 0.2 mL DMSO), 2 mg of esterase, and 0.3 M KPB with the pH being 7.5 were added to 1 mL of a reaction system; a reaction was performed for 2 h at 40 °C, and then 3 mL of absolute ethanol was added to 0.5 mL of the reaction system and fully shaken; and centrifugation was performed for 3 min at 12000 rpm, and supernatant liquid was taken for liquid phase detection, so as to detect a conversion rate and an e.e. value. Results were shown in Table 2.

**Table 2**

| Enzyme | Substrate 1 | | Substrate 2 | | Substrate 3 | | Substrate 4 | | Substrate 5 | | Substrate 6 | |
|---|---|---|---|---|---|---|---|---|---|---|---|---|
| | Conversi on rate | e. e. (% ) | Conversi on rate | e. e. (% ) | Conversi on rate | e. e. (% ) | Conversi on rate | e. e. (% ) | Conversi on rate | e. e. (% ) | Conversi on rate | e. e. (% ) |
| 1245S+L65V+T7 1V | + | ** | ++ | ** | + | *** | ++ | *** | + | ** | + | ** |
| 1245S+L65V+T7 1M | - | ** | + | *** | ++ | ** | ++ | ** | +++ | *** | +++ | *** |
| 1245S+L65V+T8 7E | +++ | *** | ++ | ** | + | ** | + | *** | ++ | *** | ++ | *** |
| 1245S+L65V+R 210Y | ++ | *** | +++ | *** | +++ | *** | + | *** | ++ | *** | + | *** |
| 1245S+L65V+K 212R | + | *** | ++ | *** | +++ | *** | + | *** | ++ | *** | + | *** |
| 1245S+L65V+P 231T | ++ | *** | ++ | *** | + | *** | + | *** | + | *** | + | *** |
| 1245S+L65V+T2 40F | + | *** | ++ | *** | + | *** * | ++ | ** | ++ | *** | ++ | *** |
| 1245S+L65V+T2 40L | + | *** | + | *** | +++ | *** | ++ | ** | + | ** | + | ** |
| 1245S+L65V+K 251T | ++ | *** | ++ | *** | ++ | *** | - | ** | ++ | *** | - | ** |
| 1245S+L65V+L3 65M | ++ | ** | + | ** | + | ** | + | ** | + | ** | ++ | *** |
| 1245S+L65V+H 262A | +++ | *** * | +++ | *** | ++ | ** | + | *** | ++ | *** | ++ | *** |
| 1245S+L65V+H 262S | + | ** | + | ** | ++ | *** | + | *** | + | ** | + | *** |
| 1245S+T71V+C 33A | - | ** | + | ** | - | ** | - | ** | + | ** | - | ** |
| 1245S+T71V+L6 5V | +++ | ** | ++ | *** | ++ | *** | + | * | + | * | ++ | ** |
| 1245S+T71V+T 87E | ++ | *** | ++ | *** | ++ | *** | + | * | +++ | * | + | *** |
| 1245S+T71V+L1 31N | + | *** | ++ | *** | ++ | *** | ++ | *** | + | ** | + | ** |
| 1245S+T71V+T 240F | ++ | * | + | * | + | ** | ++ | ** | +++ | *** | +++ | *** |
| 1245S+T71V+T 240L | ++ | * | +++ | *** * | +++ | *** * | +++ | *** * | +++ | *** * | +++ | *** * |
| 1245S+T71V+K 251T | ++ | ** | ++ | ** | ++ | ** | + | *** | ++ | *** | + | *** |
| 1245S+T71V+H 262A | +++ | ** | + | ** | +++ | ** | + | *** | ++ | *** | +++ | *** |
| 1245S+T71V+H 262S | + | ** | ++ | *** | + | *** | + | *** | + | *** | + | *** |
| 1245S+T71V+L3 65M | +++ | *** | ++ | ** | ++ | ** | ++ | ** | ++ | *** | ++ | *** |

The fold decrease or increase in activity (where the conversion rate represents enzyme activity) relative to a female parent; --- represents a decrease of 10-50 folds; -- represents a decrease of 5-10 folds; - represents a decrease of 1-5 folds; + represents an increase of 1-5 folds; ++ represents an increase of 5-10 folds;+++ represents an increase of 10-50 folds; ++++ represents an increase of more than 50 folds; and +++++ represents an increase of more than 70 folds.
* with an ee value less than 0%; ** with the ee value 0%-50%; *** with the ee value 50%-95%; and **** with the ee value greater than 95%.

### Embodiment 3

A mutation was continuously performed to improve enzyme activity and increase a product ee value, as well as increase a substrate concentration, and decrease a reaction volume.

5 mg of a substrate 1, substrate 2, substrate 3, substrate 4, substrate 5, or substrate 6 (dissolved in 0.1 mL DMSO), 1 mg of esterase, and 0.3 M KPB with the pH being 7.5 were added to 0.5 mL of a reaction system; a reaction was performed for 2 h at 40 °C, and then 3 mL of absolute ethanol was added to 0.5 mL of the reaction system and fully shaken; and centrifugation was performed for 3 min at 12000 rpm, and supernatant liquid was taken for liquid phase detection, so as to detect a conversion rate and an e.e. value. Results were shown in Table 3.

**Table 3**

| Enzyme | Substrate 1 | | Substrate 2 | | Substrate 3 | | Substrate 4 | | Substrate 5 | | Substrate 6 | |
|---|---|---|---|---|---|---|---|---|---|---|---|---|
| | Conver sion rate | e. e. (% ) | Conver sion rate | e. e. (% ) | Conver sion rate | e. e. (% ) | Conver sion rate | e. e. (% ) | Conver sion rate | e. e. (% ) | Conver sion rate | e. e. (% ) |
| 1245S+L65V+H262A +T87E | + | ** | ++ | ** | + | *** | ++ | *** | + | ** | +++ | ** |
| 1245S+L65V+H262A +R210Y | + | ** | + | *** | ++ | ** | ++ | ** | +++ | *** | +++ | *** |
| 1245S+L65V+H262A +K212R | +++ | *** | ++ | ** | + | ** | + | *** | ++ | *** | ++ | *** |
| 1245S+L65V+H262A +K212M | ++ | *** | +++ | *** | ++++ | *** | + | *** | ++ | *** | + | *** |
| 1245S+L65V+H262A +T240F | ++++ | *** * | ++ | ** | ++ | ** | + | *** | ++ | *** | ++ | *** |
| 1245S+L65V+H262A +T240L | - | ** | + | *** | + | *** | +++ | *** | + | *** | + | *** |
| 1245S+L65V+H262A +T240C | +++ | *** | ++ | ** | ++ | ** | ++ | ** | + | *** | ++ | *** |
| 1245S+L65V+H262A +S248D | ++ | *** | +++ | *** | +++ | ** | ++ | ** | + | ** | +++ | ** |
| 1245S+L65V+H262A +S248M | + | ** | ++ | ** | ++ | *** | - | ** | + | *** | - | ** |
| 1245S+L65V+H262A +K251T | + | *** | ++++ | *** * | ++++ | *** * | ++++ | *** * | ++++ | *** * | ++++ | *** * |
| 1245S+L65V+H262A +K251L | ++ | *** | ++ | ** | + | *** | + | *** | ++ | *** | ++ | *** |
| 1245S+L65V+H262A +P336C | + | ** | +++ | *** | ++ | ** | + | *** | + | ** | + | *** |
| 1245S+L65V+H262A +L365M | - | ** | + | ** | + | *** | - | ** | ++++ | ** | - | ** |
| 1245S+T71V+T240L +C33A | +++ | *** | ++ | *** | ++ | ** | + | * | + | ** | + | ** |
| 1245S+T71V+T240L +L65V | +++ | *** | +++ | ** | + | *** | ++ | *** | + | ** | + | ** |
| 1245S+T71V+T240L +T87E | + | ** | ++++ | *** * | ++++ | *** * | ++++ | *** * | ++++ | *** * | ++++ | *** * |
| 1245S+T71V+T240L +L131N | - | ** | + | ** | +++ | *** | + | *** | ++ | *** | ++ | *** |
| 1245S+T71V+T240L +S235Q | +++ | ** | ++ | *** | ++ | *** | +++ | *** | ++ | *** | + | *** |
| 1245S+T71V+T240L +K251T | +++ | *** | +++ | *** * | ++++ | *** | + | *** | ++ | *** | +++ | *** |
| 1245S+T71V+T240L +H262A | + | *** | ++ | *** | ++ | *** | + | *** | +++ | *** | + | *** |
| 1245S+T71V+T240L +H262S | ++ | ** | ++ | ** | + | ** | ++ | *** | + | ** | + | ** |
| 1245S+T71V+T240L +P336C | ++ | *** | +++ | *** * | +++ | *** * | ++ | ** | +++ | *** | +++ | *** |
| 1245S+T71V+T240L +L365M | ++ | *** | +++ | ** | ++ | ** | ++ | *** | ++ | *** | ++ | *** |

The fold decrease or increase in activity (where the conversion rate represents enzyme activity) relative to a female parent; --- represents a decrease of 10-50 folds; -- represents a decrease of 5-10 folds; - represents a decrease of 1-5 folds; + represents an increase of 1-5 folds; ++ represents an increase of 5-10 folds;+++ represents an increase of 10-50 folds; ++++ represents an increase of more than 50 folds; and +++++ represents an increase of more than 70 folds.
* with an ee value less than 0%; ** with the ee value 0%-50%; *** with the ee value 50%- 95%; and **** with the ee value greater than 95%.

### Embodiment 4

A reaction system was optimized based on reaction conditions.

5 mg of a substrate 1 (dissolved in 0.1 mL DMSO), 1 mg of esterase, and 0.3 M KPB with the pH being 7.5 were added to 0.5 mL of the reaction system. The reaction system was optimized based on reaction conditions: cosolvents DMSO (0%-25%) with different degrees of solubility, buffers (0.1 M-1 M KPB pH 7.5) with different concentrations, buffers (0.3 M KPB pH 7-8, 0.3 M Tris-HCl pH 8-9) with different pHs, and reaction temperature (20 °C-50 °C). After 2 h of the reaction, 3 mL of absolute ethanol was added to 0.5 mL of the reaction system and fully shaken; and centrifugation was performed for 3 min at 12000 rpm, and supernatant liquid was taken for liquid phase detection, so as to detect a conversion rate and an e.e. value. Results were shown in Tables 4-7.

**Table 4**

| Enzyme | Cosolvent | Substrate 1 | |
|---|---|---|---|
| | | Conversion rate | e.e. (%) |
| I245S+L65V+H262A+T240F | DMSO 0% | + | **** |
| I245S+L65V+H262A+T240F | DMSO 5% | ++ | **** |
| I245S+L65V+H262A+T240F | DMSO 10% | +++ | **** |
| I245S+L65V+H262A+T240F | DMSO 15% | +++ | **** |
| I245S+L65V+H262A+T240F | DMSO 20% | ++++ | **** |
| I245S+L65V+H262A+T240F | DMSO 25% | ++ | **** |

**Table 5**

| Enzyme | Buffer | Substrate 1 | |
|---|---|---|---|
| | | Conversion rate | e.e. (%) |
| I245S+L65V+H262A+T240F | 0.1 M KPB pH 7.5 | +++ | **** |
| I245S+L65V+H262A+T240F | 0.3 M KPB pH 7.5 | ++++ | **** |
| I245S+L65V+H262A+T240F | 0.5 M KPB pH 7.5 | +++ | **** |
| I245S+L65V+H262A+T240F | 0.7 M KPB pH 7.5 | ++ | **** |
| I245S+L65V+H262A+T240F | 1 M KPB pH 7.5 | ++ | **** |

**Table 6**

| Enzyme | Buffer | Substrate 1 | |
|---|---|---|---|
| | | Conversion rate | e.e. (%) |
| I245S+L65V+H262A+T240F | 0.3 M KPB pH 7.0 | +++ | **** |
| I245S+L65V+H262A+T240F | 0.3 M KPB pH 7.5 | ++++ | **** |
| I245S+L65V+H262A+T240F | 0.3 M KPB pH 8.0 | ++++ | **** |
| I245S+L65V+H262A+T240F | 0.3 M Tris-HCl pH 8.0 | +++++ | **** |
| I245S+L65V+H262A+T240F | 0.3 M Tris-HCl pH 8.5 | ++++ | **** |
| I245S+L65V+H262A+T240F | 0.3 M Tris-HCl pH 9.0 | +++ | **** |

**Table 7**

| Enzyme | Reaction temperature | Substrate 1 | |
|---|---|---|---|
| | | Conversion rate | e.e. (%) |
| I245S+L65V+H262A+T240F | 20 °C | + | **** |
| I245S+L65V+H262A+T240F | 25 °C | ++ | **** |
| I245S+L65V+H262A+T240F | 30 °C | +++ | **** |
| I245S+L65V+H262A+T240F | 35 °C | +++ | **** |
| I245S+L65V+H262A+T240F | 40 °C | ++++ | **** |
| I245S+L65V+H262A+T240F | 45 °C | +++ | **** |
| I245S+L65V+H262A+T240F | 50 °C | + | *** |

The fold decrease or increase in activity (where the conversion rate represents enzyme activity) relative to a female parent; --- represents a decrease of 10-50 folds; -- represents a decrease of 5-10 folds; - represents a decrease of 1-5 folds; + represents an increase of 1-5 folds; ++ represents an increase of 5-10 folds;+++ represents an increase of 10-50 folds; ++++ represents an increase of more than 50 folds; and +++++ represents an increase of more than 70 folds.
* with an ee value less than 0%; ** with the ee value 0%-50%; *** with the ee value 50% - 95%; and **** with the ee value greater than 95%.

### Embodiment 5

An amplification reaction of 1 g of a substrate was performed according to an optimized system.

The amplification reaction was performed on the substrate 1 was performed based on the optimized reaction system. 1 g of the substrate 1 (dissolved in 4 mL DMSO), 50 mg of esterase (I245S+L65V+H262A+T240F), and 0.3 M Tris-HCl with the pH being 8.0 were added to 20 mL of the reaction system. The reaction was performed at 40 °C, a reaction time was tracked for sampling and detection, and the pH was adjusted to about 8.0, until a substrate of one of configurations was completely converted. Sampling and detection: 3 mL of absolute ethanol was added to 100 µL of the reaction system and fully shaken; and centrifugation was performed for 3 min at 12000 rpm, and supernatant liquid was taken for liquid phase detection, so as to detect a conversion rate and an e.e. value. Results were shown in Table 8.

**Table 8**

| Enzyme | Time | Substrate 1 | |
|---|---|---|---|
| | | Conversion rate | e.e. (%) |
| I245S+L65V+H262A+T240F | 0.5 h | + | **** |
| | 1 h | + | **** |
| | 1.5 h | ++ | **** |
| | 2 h | ++ | **** |
| | 2.5 h | ++ | **** |
| | 3 h | +++ | **** |
| | 3.5 h | +++ | **** |
| | 4 h | +++ | **** |

A conversion rate gradually increased with the reaction time. + represents that the conversion rate was 0-10%; ++ represents that the conversion rate was 10-45%; and +++ represents that the conversion rate was 45-50%.
* with an ee value less than 0%; ** with the ee value 0%- 50%; *** with the ee value 50% - 95%; and **** with the ee value greater than 95%.

### Embodiment 6

Beneficial mutation sites were further combined to further increase a substrate concentration, thereby reducing a reaction volume.

10 mg of a substrate 2, substrate 3, substrate 4, substrate 5, or substrate 6 (dissolved in 0.1 mL DMSO), 1 mg of esterase, and 0.3 M KPB with the pH being 7.5 were added to 0.5 mL of a reaction system; a reaction was performed for 2 h at 40 °C, and then 3 mL of absolute ethanol was added to 0.5 mL of the reaction system and fully shaken; and centrifugation was performed for 3 min at 12000 rpm, and supernatant liquid was taken for liquid phase detection, so as to detect a conversion rate and an e.e. value. Results were shown in Table 9.

**Table 9**

| Enzyme | Substrate 2 | | Substrate 3 | | Substrate 4 | | Substrate 5 | | Substrate 6 | |
|---|---|---|---|---|---|---|---|---|---|---|
| | Convers ion rate | e. e. (% ) | Convers ion rate | e. e. (% ) | Convers ion rate | e. e. (% ) | Convers ion rate | e. e. (% ) | Convers ion rate | e. e. (% ) |
| I245S+T71V+T240F+L131N+S48N | ++ | *** | ++ | *** | ++ | *** | ++ | ** | ++ | ** |
| I245S+T71V+T240L+K251T+L131N | +++ | *** | +++ | ** | ++ | *** | +++ | *** | +++ | *** |
| I245S+T71V+T240L+K251T+R210Y | ++ | *** | ++++ | *** * | + | *** | ++ | *** | ++ | *** |
| 1245S+T71V+T240L+K251T+K212R | ++ | *** * | ++ | *** * | +++ | *** * | +++ | *** | + | *** |
| 1245S+T71V+T240L+K251T+K212M | +++++ | *** | ++++ | *** | ++ | *** | ++ | *** | +++ | *** * |
| 1245S+T71V+T240L+K251T+S235Q | +++ | ** | ++ | *** | ++ | *** | ++ | ** | + | ** |
| 1245S+T71V+T240L+K251T+H262A | ++++ | *** * | +++++ | *** | +++ | *** * | +++ | *** | +++ | *** |
| 1245S+T71V+T240L+K251T+H262S | +++ | *** | ++++ | ** | + | *** | ++++ | *** | ++++ | *** |
| 1245S+T71V+T240L+K251T+P336C | +++ | *** * | ++ | *** * | +++ | *** | ++ | *** | + | *** |
| I245S+T71V+T240L+K251T+L365M | + | ** | +++ | *** * | +++ | *** | ++ | *** | + | *** * |
| 1245S+T71V+T240L+T87E+L65V | +++ | *** | +++ | ** | + | *** | + | *** | + | *** |
| I245S+T71V+T240L+T87E+L65V+R 210Y | ++ | ** | ++ | ** | ++ | ** | +++ | *** | ++ | *** |
| 1245S+T71V+T240L+T87E+L65V+K 212M | +++++ | *** * | +++++ | *** * | +++++ | *** * | +++++ | *** * | +++++ | *** * |
| 1245S+T71V+T240L+T87E+L65V+K 251T | ++ | *** | +++++ | *** | - | *** | + | *** | - | ** |
| 1245S+T71V+T240L+T87E+L65V+H 262A | +++++ | *** | ++ | ** | + | *** | ++++ | ** | ++ | *** |
| 1245S+T71V+T240L+T87E+L65V+H 262S | ++ | *** * | ++ | ** | +++ | *** | ++ | *** | ++ | *** |
| 1245S+T71V+T240L+T87E+L65V+H 262C | ++ | ** | ++ | *** * | ++ | *** | +++ | ** | ++++ | *** |
| 1245S+T71V+T240L+T87E+L65V+K 251T+K212M | +++++ | *** * | ++++ | ** | - | *** | ++++ | *** * | - | *** * |
| 1245S+T71V+T240L+T87E+L65V+K 251T+H262A | ++ | ** | ++ | *** * | ++++ | *** * | + | ** | + | *** |
| 1245S+T71V+T240L+T87E+L65V+K 251T+L365M | ++++ | *** * | +++ | *** | + | *** | +++ | *** | ++++ | *** |
| I245S+L65V+H262A+T240F+L131S | +++ | *** * | +++ | ** | +++ | *** | ++ | ** | + | *** * |
| I245S+L65V+H262A+T240F+L131H | +++ | *** | ++++ | *** * | ++ | ** | +++ | *** | +++ | *** |
| I245S+L65V+H262A+T240F+L131N | ++ | *** * | +++ | *** | +++ | *** * | ++ | *** | +++ | *** |
| I245S+L65V+H262A+T240F+R210Y | ++++ | *** * | +++ | ** | + | *** | ++ | *** | + | *** |
| I245S+L65V+H262A+T240F+K212M | ++ | *** | ++ | *** * | ++++ | *** * | ++ | *** * | ++++ | *** * |
| I245S+L65V+H262A+T240F+P231T | +++ | *** * | ++ | *** | + | *** | + | *** | ++ | *** |
| I245S+L65V+H262A+T240F+K251T | +++ | *** * | +++++ | *** | ++++ | *** * | ++++ | *** | ++ | *** * |
| 1245S+L65V+H262A+T240F+L365M | +++ | *** * | +++ | *** | ++ | ** | + | ** | +++ | *** |

The fold decrease or increase in activity (where the conversion rate represents enzyme activity) relative to a female parent; --- represents a decrease of 10-50 folds; -- represents a decrease of 5-10 folds; - represents a decrease of 1-5 folds; + represents an increase of 1-5 folds; ++ represents an increase of 5-10 folds;+++ represents an increase of 10-50 folds; ++++ represents an increase of more than 50 folds; and +++++ represents an increase of more than 70 folds.
* with an ee value less than 0%; ** with the ee value 0% - 50%; *** with the ee value 50%-95%; and **** with the ee value greater than 95%.

### Embodiment 7

A reaction system was optimized based on reaction conditions.

5 mg of a substrate 2 (dissolved in 0.1 mL DMSO), 0.5 mg of esterase, and 0.3 M KPB with the pH being 7.5 were added to 0.5 mL of the reaction system. The reaction system was optimized based on reaction conditions: cosolvents DMSO (0%-25%) with different degrees of solubility, buffers (0.1 M-1 M KPB pH 7.5) with different concentrations, buffers (0.3 M KPB pH 7-8, 0.3 M Tris-HCl pH 8-9) with different pHs, and reaction temperature (20 °C-50 °C). After 2 h of the reaction, 3 mL of absolute ethanol was added to 0.5 mL of the reaction system and fully shaken; and centrifugation was performed for 3 min at 12000 rpm, and supernatant liquid was taken for liquid phase detection, so as to detect a conversion rate and an e.e. value. Results were shown in Tables 10-13.

**Table 10**

| Enzyme | Cosolven t | Substrate 2 | | Substrate 4 | |
|---|---|---|---|---|---|
| | | Conversio n rate | e.e . (%) | Conversio n rate | e.e (%) |
| I245S+T71V+T240L+T87E+L65V+K251T+K212 M | DMSO 0% | + | **** | + | **** |
| I245S+T71V+T240L+T87E+L65V+K251T+K212 M | DMSO 5% | ++ | **** | ++ | **** |
| I245S+T71V+T240L+T87E+L65V+K251T+K212 M | DMSO 10% | +++ | **** | +++ | **** |
| I245S+T71V+T240L+T87E+L65V+K251T+K212 | DMSO | ++++ | **** | ++++ | **** |
| M | 15% | | | | |
| I245S+T71V+T240L+T87E+L65V+K251T+K212 M | DMSO 20% | +++++ | **** | +++++ | **** |
| I245S+T71V+T240L+T87E+L65V+K251T+K212 M | DMSO 25% | +++ | **** | +++ | **** |

**Table 11**

| Enzyme | Buffer | Substrate 2 | | Substrate 4 | |
|---|---|---|---|---|---|
| | | Conversion rate | e.e. (%) | Conversio n rate | e.e. (%) |
| I245S+T71V+T240L+T87E+L65V+K251T+K 212M | 0.1 M KPB pH 7.5 | ++++ | **** | ++++ | **** |
| I245S+T71V+T240L+T87E+L65V+K251T+K 212M | 0.3 M KPB pH 7.5 | +++++ | **** | +++++ | **** |
| I245S+T71V+T240L+T87E+L65V+K251T+K 212M | 0.5 M KPB pH 7.5 | ++++ | **** | ++++ | **** |
| I245S+T71V+T240L+T87E+L65V+K251T+K 212M | 0.7 M KPB pH 7.5 | ++++ | **** | ++++ | **** |
| I245S+T71V+T240L+T87E+L65V+K251T+K 212M | 1 M KPB pH 7.5 | +++ | **** | +++ | **** |

**Table 12**

| Enzyme | Buffer | Substrate 2 | | Substrate 4 | |
|---|---|---|---|---|---|
| | | Conversio n rate | e.e . (%) | Conversio n rate | e.e (%) |
| I245S+T71V+T240L+T87E+L65V+K251T +K212M | 0.3 M KPB pH 7.0 | ++++ | **** | ++++ | **** |
| I245S+T71V+T240L+T87E+L65V+K251T +K212M | 0.3 M KPB pH 7.5 | +++++ | **** | +++++ | **** |
| I245S+T71V+T240L+T87E+L65V+K251T +K212M | 0.3 M KPB pH 8.0 | +++++ | **** | +++++ | **** |
| I245S+T71V+T240L+T87E+L65V+K251T +K212M | 0.3 M Tris-HCl pH 8.0 | ++++++ | **** | ++++++ | **** |
| I245S+T71V+T240L+T87E+L65V+K251T +K212M | 0.3 M Tris-HCl pH 8.5 | +++++ | **** | +++++ | **** |
| I245S+T71V+T240L+T87E+L65V+K251T +K212M | 0.3 M Tris-HCl pH 9.0 | ++++ | **** | ++++ | **** |

**Table 13**

| Enzyme | Reaction temperature | Substrate 2 | | Substrate 4 | |
|---|---|---|---|---|---|
| | | Conversio n rate | e.e . (%) | Conversio n rate | e.e (%) |
| I245S+T71V+T240L+T87E+L65V+K251T+K 212M | 20 °C | + | **** | + | **** |
| I245S+T71V+T240L+T87E+L65V+K251T+K 212M | 25 °C | ++ | **** | ++ | **** |
| I245S+T71V+T240L+T87E+L65V+K251T+K 212M | 30 °C | +++ | **** | +++ | **** |
| I245S+T71V+T240L+T87E+L65V+K251T+K 212M | 35 °C | ++++ | **** | ++++ | **** |
| I245S+T71V+T240L+T87E+L65V+K251T+K 212M | 40 °C | +++++ | **** | +++++ | **** |
| I245S+T71V+T240L+T87E+L65V+K251T+K 212M | 45 °C | ++++ | **** | ++++ | **** |
| I245S+T71V+T240L+T87E+L65V+K251T+K 212M | 50 °C | ++ | **** | ++ | **** |

The fold decrease or increase in activity (where the conversion rate represents enzyme activity) relative to a female parent; --- represents a decrease of 10-50 folds; -- represents a decrease of 5-10 folds; - represents a decrease of 1-5 folds; + represents an increase of 1-5 folds; ++ represents an increase of 5-10 folds;+++ represents an increase of 10-50 folds; ++++ represents an increase of more than 50 folds; +++++ represents an increase of more than 70 folds; and ++++++ represents an increase of more than 100 folds.
* with an ee value less than 0%; ** with the ee value 0%- 50%; *** with the ee value 50%- 95%; and **** with the ee value greater than 95%.

### Embodiment 8

An amplification reaction of 1 g of a substrate was performed according to an optimized system.

1 g of the substrate 2 (dissolved in 4 mL DMSO), 25 mg of esterase (I245S+T71V+T240L+T87E+L65V+K251T+K212M), and 0.3 M Tris-HCl with the pH being 8.0 were added to 20 mL of the reaction system. The reaction was performed at 40 °C, a reaction time was tracked for sampling and detection, and the pH was adjusted to about 8.0, until a substrate of one of configurations was completely converted. Sampling and detection: 3 mL of absolute ethanol was added to 100 µL of the reaction system and fully shaken; and centrifugation was performed for 3 min at 12000 rpm, and supernatant liquid was taken for liquid phase detection, so as to detect a conversion rate and an e.e. value. Results were shown in Table 14.

**Table 14**

| Enzyme | Time | Substrate 2 | |
|---|---|---|---|
| | | Conversion rate | e.e. (%) |
| | 0.5 h | + | **** |
| | 1 h | ++ | **** |
| I245S+T71V+T240L+T87E+L65V+K251T+K212M | 1.5 h | ++ | **** |
| | 2 h | ++ | **** |
| | 2.5 h | ++ | **** |
| | 3 h | +++ | **** |
| | 3.5 h | +++ | **** |
| | 4 h | +++ | **** |

A conversion rate gradually increased with the reaction time. + represents that the conversion rate was 0-10%; ++ represents that the conversion rate was 10-45%; and +++ represents that the conversion rate was 45%-50%.
* with an ee value less than 0%; ** with the ee value 0%-50%; *** with the ee value 50%-95%; and **** with the ee value greater than 95%.

### Embodiment 9

An amplification reaction of 1 g of a substrate was performed according to an optimized system.

1 g of the substrate 4 (dissolved in 4 mL DMSO), 25 mg of esterase (I245S+T71V+T240L+T87E+L65V+K251T+K212M), and 0.3 M Tris-HCl with the pH being 8.0 were added to 20 mL of the reaction system. The reaction was performed at 40 °C, a reaction time was tracked for sampling and detection, and the pH was adjusted to about 8.0, until a substrate of one of configurations was completely converted. Sampling and detection: 3 mL of absolute ethanol was added to 100 µL of the reaction system and fully shaken; and centrifugation was performed for 3 min at 12000 rpm, and supernatant liquid was taken for liquid phase detection, so as to detect a conversion rate and an e.e. value. Results were shown in Table 15.

**Table 15**

| Enzyme | Time | Substrate 4 | |
|---|---|---|---|
| | | Conversion rate | e.e. (%) |
| | 0.5 h | + | **** |
| | 1 h | + | **** |
| I245S+T71V+T240L+T87E+L65V+K251T+K212M | 1.5 h | ++ | **** |
| | 2 h | ++ | **** |
| | 2.5 h | ++ | **** |
| | 3 h | +++ | **** |
| | 3.5 h | +++ | **** |
| | 4 h | +++ | **** |

A conversion rate gradually increased with the reaction time. + represents that the conversion rate was 0-10%; ++ represents that the conversion rate was 10-45%; and +++ represents that the conversion rate was 45%-50%.
* with an ee value less than 0%; ** with the ee value 0%-50%; *** with the ee value 50%-95%; and **** with the ee value greater than 95%.

The above are only the preferred embodiments of the present invention and are not intended to limit the present invention. For those skilled in the art, the present invention may have various modifications and variations. Any modifications, equivalent replacements, improvements and the like made within the spirit and principle of the present invention all fall within the scope of protection of the present invention.

## Claims

1. An esterase mutant, wherein the esterase mutant having the sequence shown in SEQ ID NO. 1 with amino acid mutation, wherein the mutation comprises I245S; or the esterase mutant comprises an I245S mutation and has more than 95% identity with SEQ ID NO:1.

2. The esterase mutant according to claim 1, wherein the mutation also comprises any one or more of the followings: C33A, A46G, S48N, L65V, T71V, T71M, T87E, L131S, L131N, L131H, R184E, R210Y, K212R, K212M, S230Q, P231T, S235Q, T240F, T240L, T240C, S248D, S248M, K251T, K251L, H262A, H262S, H262C, P336C or L365M.

3. The esterase mutant according to claim 1, wherein the mutation comprises any one of the following combined mutations: I245S+C33A, I245S+A46G, I245S+S48N, I245S+L65V, I245S+T71V, I245S+T71M, I245S+T87E, I245S+L131N, I245S+R184E, I245S+R210Y, I245S+K212R, I245S+S230Q, I245S+P231T, I245S+T240F, I245S+T240L, I245S+S248D, I245S+K251T, I245S+K251L, I245S+H262A, I245S+H262S, I245S+P336C, I245S+L365M, I245S+L65V+T71V, I245S+L65V+T71M, I245S+L65V+T87E, I245S+L65V+R210Y, I245S+L65V+K212R, I245S+L65V+P231T, I245S+L65V+T240F, I245S+L65V+T240L, I245S+L65V+K251T, I245S+L65V+L365M, I245S+L65V+H262A, I245S+L65V+H262S, I245S+T71V+C33A, I245S+T71V+L65V, I245S+T71V+T87E, I245S+T71V+L131N, I245S+T71V+T240F, I245S+T71V+T240L, I245S+T71V+K251T, I245S+T71V+H262A, I245S+T71V+H262S, I245S+T71V+L365M, I245S+L65V+H262A+T87E, 1245S+L65V+H262A+R210Y, I245S+L65V+H262A+K212R, I245S+L65V+H262A+K212M, I245S+L65V+H262A+T240F, I245S+L65V+H262A+T240L, I245S+L65V+H262A+T240C, I245S+L65V+H262A+S248D, I245S+L65V+H262A+S248M, I245S+L65V+H262A+K251T, I245S+L65V+H262A+K251L, I245S+L65V+H262A+P336C, I245S+L65V+H262A+L365M, I245S+T71V+T240L+C33A, 1245S+T71V+T240L+L65V, I245S+T71V+T240L+T87E, 1245S+T71V+T240L+L131N, I245S+T71V+T240L+S235Q, I245S+T71V+T240L+K251T, 1245S+T71V+T240L+H262A, I245S+T71V+T240L+H262S, I245S+T71V+T240L+P336C, 1245S+T71V+T240L+L365M, I245S+T71V+T240F+L131N+S48N, I245S+T71V+T240L+K251T+L131N, I245S+T71V+T240L+K251T+R210Y, I245S+T71V+T240L+K251T+K212R, I245S+T71V+T240L+K251T+K212M, I245S+T71V+T240L+K251T+S235Q, I245S+T71V+T240L+K251T+H262A, I245S+T71V+T240L+K251T+H262S, I245S+T71V+T240L+K251T+P336C, I245S+T71V+T240L+K251T+L365M, I245S+T71V+T240L+T87E+L65V, I245S+T71V+T240L+T87E+L65V+R210Y, I245S+T71V+T240L+T87E+L65V+K212M, I245S+T71V+T240L+T87E+L65V+K251T, I245S+T71V+T240L+T87E+L65V+H262A, I245S+T71V+T240L+T87E+L65V+H262S, 1245S+T71V+T240L+T87E+L65V+H262C, 1245S+T71V+T240L+T87E+L65V+K251T+K212M, I245S+T71V+T240L+T87E+L65V+K251T+H262A, I245S+T71V+T240L+T87E+L65V+K251T+L365M, 1245S+L65V+H262A+T240F+L131S, I245S+L65V+H262A+T240F+L131H, I245S+L65V+H262A+T240F+L131N, I245S+L65V+H262A+T240F+R210Y, I245S+L65V+H262A+T240F+K212M, I245S+L65V+H262A+T240F+P231T, I245S+L65V+H262A+T240F+K251T or I245S+L65V+H262A+T240F+L365M.

4. A DNA molecule, wherein the DNA molecule encodes the esterase mutant according to any one of claims 1 to 3.

5. A recombinant plasmid, wherein the recombinant plasmid contains the DNA molecule according to claim 4.

6. The recombinant plasmid according to claim 5, wherein the recombinant plasmid is pET-22a (+), pET-22b (+), pET-3a (+), pET-3d (+), pET-11a (+), pET-12a (+), pET-14b, pET-15b (+), pET-16b (+), pET-17b (+), pET-19b (+), pET-20b (+), pET-21a (+), pET-23a (+), pET-23b (+), pET-24a (+), pET-25b (+), pET-26b (+), pET-27b (+), pET-28a (+), pET-29a (+), pET-30a (+), pET-31b (+), pET-32a (+), pET-35b (+), pET-38b (+), pET-39b (+), pET-40b (+), pET-41a (+), pET-41b (+), pET-42a (+), pET-43a (+), pET-43b (+), pET-44a (+), pET-49b (+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-8, pUC-18 or pUC-19.

7. A host cell, wherein the host cell comprises the recombinant plasmid according to claim 5 or 6.

8. The host cell according to claim 7, wherein the host cell comprises a prokaryotic cell or a eukaryotic cell; preferably, the prokaryotic cell is *Escherichia coli* BL21 cells or *Escherichia coli* DH5α competent cell, wherein the eukaryotic cell is yeast.

9. A method for producing a chiral acid comprising the step of performing a catalytic reaction of ester compounds using an esterase, wherein the esterase is the esterase mutant according to any one of claims 1 to 3.

10. The method according to claim 9, wherein the ester compound is 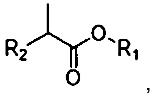 the reaction product is 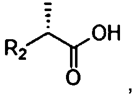 wherein R1 represents -CH₃, -CH₂CH₃, -CH₂CH₂CH₃ or -CH₂CH₃CH₃; R2 represents one or more of a halogen atom, a nitrogen atom, an oxygen atom, a carbon chain, a heterocycle or a naphthalene ring having different substituents of a halogen atom, a nitrogen atom, an oxygen atom, and a carbon chain; preferably, the ester compound is

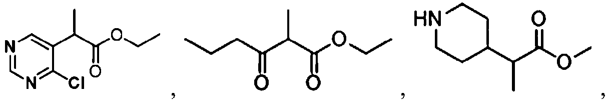

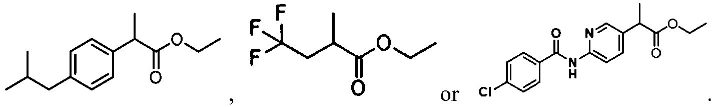

11. The method according to claim 10, wherein the catalytic reaction of the esterase mutant has a pH of 7.0 to 9.0 and a reaction temperature of 20°C to 50°C.
